# EUROPEAN PATENT APPLICATION

(11) **EP 4 035 704 A1**
(43) Date of publication of application: **03.08.2022**
(21) Application number: 21154254.3
(22) Date of filing: 29.01.2021
(51) Int. Cl.: A61M 1/16, A61M 1/28

(54) **APPARATUS FOR DIALYSIS AND A HEATER CONTROL CIRCUIT FOR A DIALYSIS MACHINE**

(71) Applicant: Bellco S.r.l., 41037 Mirandola (MO) (IT)
(72) Inventor: MARRA, Antonia Giuseppe, 41037 Modena (IT)
(74) Representative: Maschio, Antonio

(57) **Abstract**

A solid-state relay is used to couple a power supply to a heater for dialysate in a dialysis machine. A solid-state relay avoids the production of annoying noise that results from the use of conventional relays. In a preferred embodiment, a solid-state relay is used which switches at a zero-crossing point of the power waveform applied. This results in less interference being created by the heater. The heater itself in a preferred form is one with low inductance and is most preferred as a purely resistive heater.

## Description

### FIELD OF THE INVENTION

This invention relates to medical apparatus for dialysis.

### BACKGROUND

One type of dialysis for the treatment of kidney failure is peritoneal dialysis (PD) which uses the lining of a patient's abdomen, the peritoneum, to filter blood while it remains inside the body. Peritoneal Dialysis is an effective way to treat patients with End Stage Renal Disease and acute patients with renal failure. The patient is provided with a catheter that includes a transfer set for selective connection to a dialysis solution or a drain bag. A dialysis solution ('dialysate' or 'peritoneal dialysis fluid') comprising water with other electrolytes is warmed to body temperature and then introduced into the peritoneal cavity of the patient via the catheter. The solution remains here for a period of time required to clean the blood of waste products, toxins and excess fluid and these are then removed from the body to leave the right amounts of electrolytes and nutrients in the blood. The machine or 'cycler' may repeat the exchange process a number of times (cycles), as required for a particular patient.

Conventionally dialysate consists of purified water, glucose and electrolytes, with the concentration of electrolytes resembling that which occurs naturally in the blood. It is prepared according to an individual patient's needs to help regulate their electrolyte and acid-base balance and remove metabolic waste products. The dialysate components are normally shipped in the form of concentrates, with basic and acidic concentrates being prepared separately. The basic concentrate is usually supplied as a dry concentrate consisting of pure sodium bicarbonate which is made up into a saturated solution and the acidic concentrate is usually provided as a liquid concentrate. If it is provided as a dry concentrate, the entire concentrate must be dissolved before being diluted to form the dialysate. This requires a large volume of liquid and multiple components for providing the peritoneal dialysis fluid, increasing the time required for production of the fluid. Concentrate may also be wasted during the production of the correct peritoneal dialysis fluid and difficulties may be encountered in providing the correct concentration of electrolytes in the fluid for a particular patient. In order to make dialysis more convenient for patients, it has been proposed to provide dialysis machines at a patient's home. This creates a number of challenges for present designs as, for the home environment, electronic equipment must conform to certain standards to prevent undue electrical interference. Examples of such standards are IEC 6000-3-2 (Harmonics) and IEC 61000-3-3 (Voltage fluctuations and Flicker).

As described above, in dialysis it is necessary to introduce the dialysate into the peritoneal cavity of the patient via the catheter. Before this is done, the dialysate is heated to body temperature by the dialysis machine. Conventional heating elements may comprise wound coils which have a significant inductance which when connected to an alternating power supply will result in significant interference and voltage fluctuations and flicker contrary to the above-mentioned standards due to the induced reactance.

In a first aspect of the invention, the invention provides a dialysis machine provided with a heater driven by solid state relay.

In a preferred form, the heater is a low inductance/ low reactance heater since this will further reduce harmonic or other interference effects.

### Summary of The Invention

In a first aspect of the invention there is provided a heater control circuit for use in a dialysis machine comprising; a heater for, in use, heating a dialysate, a solid-state relay operable to couple a power supply to the heater when the power supplied by the power supply is at a substantially zero crossing point.

Preferably the heater is a low inductance or low reactance heater and most preferred is a heater that is a substantially purely resistive heater.

Preferably, the solid-state relay is a synchronous solid-state relay as this will avoid the need to provide additional circuitry to detect zero-crossing however in some embodiments other types of solid state relays may be provided in which case a zero-crossing point detector is provided to detect when the power supplied by the power supply is at or near the zero crossing point and to provide a switching signal to the soldi state relay to couple the power to the heater.

### Brief Description Of The Drawings

Figure 1 shows a dialysis machine in accordance with the invention being used to perform dialysis on a patient;
Figure 2 shows a heater control circuit of the dialysis machine of figure 1 in accordance with a first embodiment of the invention; and
Figure 3 shows a second embodiment of the heater control circuit.

### Detailed Description of Preferred Embodiments of the Invention

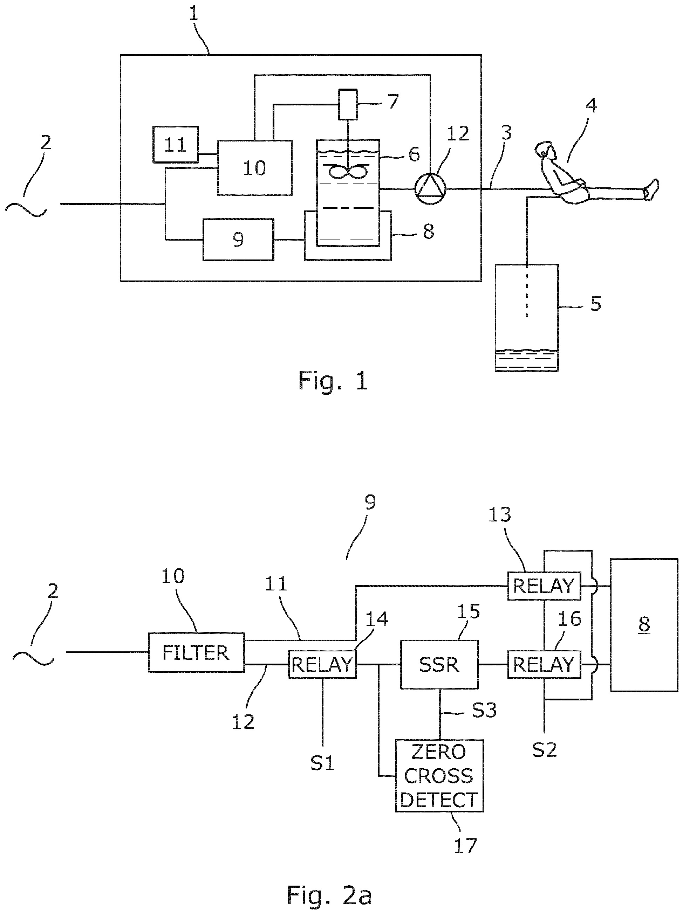

As is shown in figure 1, a dialysis machine 1 is connected to a mains power supply 2 to supply dialysate via a tube and cannula 3 into a patient 4. The dialysate enters the patient and remains there until dialysis is complete with the used dialysate being collected in a waste bag 5 which may then be discarded.

Within the dialysis machine 1 is located a dialysate tank 6 holding a supply of dialysate which is mixed by a mixer and paddle 7. The dialysate may be provided ready mixed or as a powder which is mixed with water purified from a domestic supply. In this case the solution is provided in a cassette to be loaded into the tank 6. It will be appreciated that the dialysate held in the tank will be at a temperature which is dependent on the ambient conditions. For it to be used in a patient, the temperature needs to be raised to that of typical body temperature. In order to do this, a heater 8 is provided in good thermal contact with the tank 6 to heat the dialysate which is controlled by a heater control section 9. A dialysis controller 10 is connected to all elements of the dialysis machine by a control and data bus architecture to operate in accordance with a software program held in memory 11. A pump and valve 12 is controlled by the processor 10 to administer the dialysate to the patient in accordance with the desired dialysis.

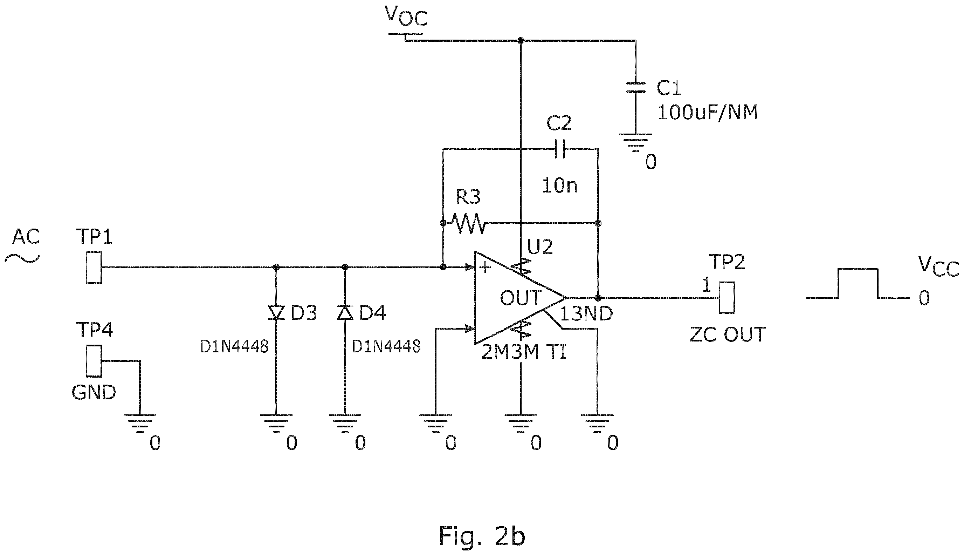

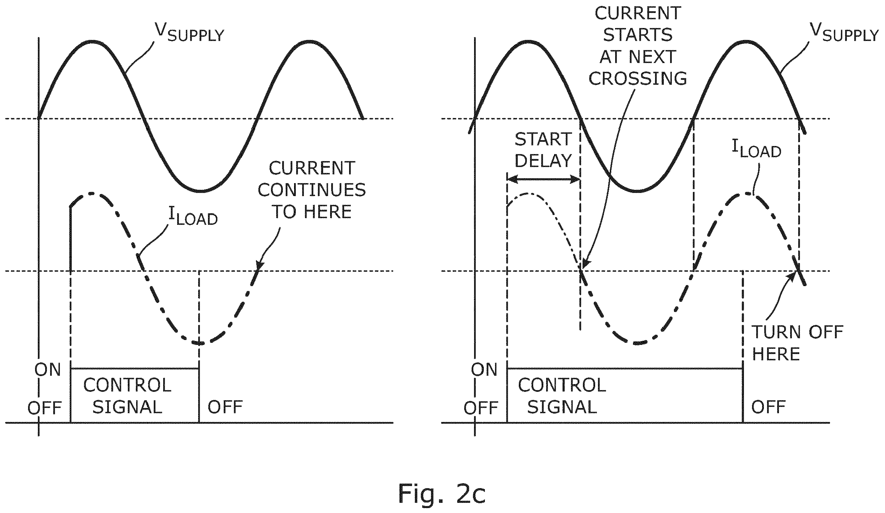

The way in which the heater 8 and the heater control section 9 is configured in accordance with a first embodiment of the invention will now be described with reference to figure 2. In this embodiment a zero-crossing detector is used to control a solid-state relay. In a later embodiment, the zero-crossing circuit is not required as a synchronous solid-state relay is used which switches at zero crossing points.

As shown in figure 2, the heater control section 9 is configured from a filter section 10 coupled to the applied AC power supply. This filters out interference present on the AC supply. The filtered supply is then coupled to a neutral and live rail 11 and 12. The neutral rail 11 is coupled via a relay 13 to the heater 8. The relay 13 is operable to connect the neutral rail to the heater upon application of a switch signal S2 received from processor 10.

The live rail 12 is coupled by a relay 14 to a solid-state relay 15. This is controlled by a switch signal S1 received from processor 10.

In this embodiment the solid-state relay 15 is arranged to switch in accordance with an input signal S3 which is provided from a zero-crossing detector 17.

The zero-crossing detector 17 is a processor which monitors the input live waveform to determine when the waveform is close to or at a zero point. It then provides the signal S3 to the SSR 15 to connect the live rail to a relay 16 and hence when that relay is on an on state to the heater 8.

The relay 16 on the live rail is switched to an on-state when it receives the signal S2. Thus, for safety, it will be seen that both the live and the neutral rails are switchable to connect the supply to the heater 8.

Accordingly, switching of the solid-state relay 15 takes place when the input ac waveform is at a zero-crossing point and hence the heater is switched at that point. As a result, any inductance of the heater 8 that would result in the creation of harmonic interference is mitigated.

A zero-crossing circuit suitable for use in the first embodiment is shown in figure 2a. In this a LM311 Differential Comparator IC is configured to compare an AC input signal at TP1 with a zero voltage value held at TP4. When the input at TP1 matches TP4 then the TP1 is zero that is to say a zero crossing is detected, the output TP2 provides a pulse rising from zero to +Vcc.

The LM311 IC has a rapid response time. However, there will still be a delay of about 100 micro seconds between a zero crossing point being reached and the output pulse being generated at TP2.

In order to cater for this delay, the switching on of the heater may be arranged to be delayed by one waveform as is illustrated by figure 2 c. This may be done by the use of suitable timers and delay circuits which will be readily apparent to a person skilled in the art which are programmed with a suitable delay value for the AC waveform of the particular supply.

Further mitigation is provided by selecting a preferred heater type which is low inductance or as close to purely resistive as possible. Examples of such heaters are FIREROD ^{™} cartridge immersion or FLUENT^{®} in-line heater available from Watlow Electric Manufacturing Company . The heater 8 may be a low inductance/ reactance heater or purely resistive.

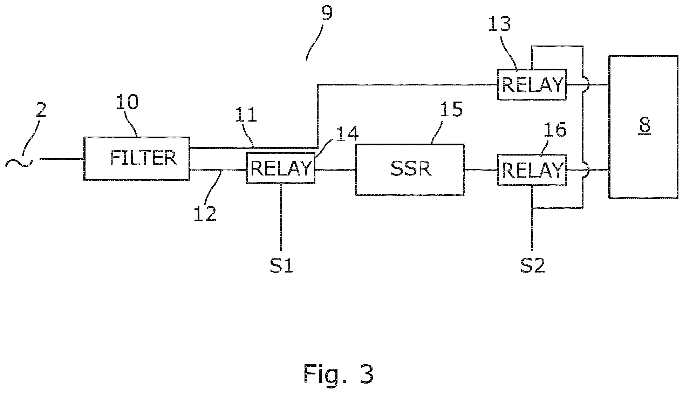

Examples of suitable solid state relays are Solitron^{™} mini RJ18 available from Carlo Gavazzi Group. Figure 3 shows a further embodiment which is preferred since it reduces the component count further over the embodiment shown in figure 2. Like components share the same reference numerals as the embodiment of figure 2.

As is shown in figure 3, the solid-state relay is a synchronous solid-state relay. A suitable synchronous solid-state relay that may be used is D2425 zero crossing solid state relay available from Crydom. It is an inherent property of this type of relay, that switching of the relay occurs during the waveform zero crossing point hence reducing interference as before. Accordingly, the distinct zero-crossing circuit 17 of the embodiment shown in figure 2 is not required.

In alternatives to the described embodiments, the relays 13, 14 and 16 may be dispensed with and the heater switching on being controlled by the solid-state relay alone.

It will be appreciated that in the embodiments, the temperature of the dialysate will be monitored by the processor and the heater controlled to ensure that the dialysate is maintained at the desired temperature.

## Claims

**1.** A heater control circuit for use in a dialysis machine comprising:
a heater for, in use, heating a dialysate;
a solid-state relay operable to couple a power supply to the heater when the power supplied by the power supply is at a substantially zero crossing point.

**2.** A heater control circuit as claimed in claim 1 wherein the heater is a low inductance or low reactance heater.

**3.** A heater control circuit as claimed in claim 2 wherein the heater is a substantially purely resistive heater.

**4.** A heater control circuit as claimed in claim 1 wherein the solid-state relay is a synchronous solid-state relay.

**5.** A heater control circuit as claimed in any one of claims 1 to 3 further comprising a zero-crossing point detector to detect when the power supplied by the power supply is at or near the zero crossing point and to provide a switching signal to the solid state relay to couple the power to the heater.

**7.** A heater control circuit as claimed in claim 5 wherein the switching signal is delayed to cater for a response time for the zero-crossing point detector.

**8.** A heater control circuit as claimed in claim 7 wherein the switching signal is delayed to coincide with a subsequent zero crossing point.

**9.** A dialysis machine comprising a heater control circuit as claimed in any preceding claim.
